# EUROPEAN PATENT SPECIFICATION

(11) **EP 3 146 983 B1**
(45) Date of publication and mention of the grant of the patent: **18.11.2020**
(21) Application number: 15186348.7
(22) Date of filing: 22.09.2015
(51) Int. Cl.: A61L 2/14, A23L 3/3409, A23L 3/3454, A61L 2/18

(54) **COMBINATION METHOD FOR CLEANING, DECONTAMINATION, DISINFECTION AND STERILIZATION OF OBJECTS**
KOMBINATIONSVERFAHREN ZUR REINIGUNG, DEKONTAMINATION, DESINFEKTION UND STERILISATION VON OBJEKTEN
PROCÉDÉ DE COMBINAISON POUR LE NETTOYAGE, LA DÉCONTAMINATION, DÉSINFECTION ET STÉRILISATION D'OBJETS

(43) Date of publication of application: 29.03.2017
(73) Proprietor: Leibniz-Institut für Plasmaforschung und Technologie e.V., 17489 Greifswald (DE)
(72) Inventor: EHLBECK, Jörg, 17498 Hinrichshagen (DE); STACHOWIAK, Jörg, 17489 Greifswald (DE); SCHNABEL, Uta, 17491 Greifswald (DE); ANDRASCH, Mathias, 17439 Stralsund (DE)
(74) Representative: Schulz Junghans Patentanwälte PartGmbB

(56) References cited:
- WO-A1-2007/048806
- WO-A1-2011/138463
- DE-A1-102013 109 777
- US-A1- 2010 006 121
- US-A1- 2013 142 694
- US-A1- 2014 100 277
- US-A1- 2015 038 584
- US-A1- 2015 110 672

## Description

The invention relates to a method for cleaning and decontaminating and/or disinfecting and/or sterilizing objects, subsequently using a plasma-generated sterilizing liquid and a treatment gas.

### Background of the invention

Known sterilization methods are autoclaving, irradiation with ionizing rays, gas sterilization with ethylene oxide, hydrogen peroxide sterilization and plasma sterilization.

Significant disadvantages are toxicity (ethylene oxide), long evaporation times (hydrogen peroxide), major requirements on equipment and safety (ionizing rays), missing suitability for heat sensitive items (autoclaving).

The sterilizing effect of a plasma depends on destruction of the germs and biomolecules by different mechanisms, for instance UV and VUV radiation, which destroy DNA.

Specific disadvantages of known plasma sterilization methods are the need to store chemical substances for the formation of the active gases, long treatment times and limited suitability for the treatment of heat-sensitive items and complex structures, since the ability of the plasma to penetrate into narrow gaps and lumens is not very good.

WO 2011/138463 A1 describes a plasma-sterilization method, in which the process of ionization takes place spatially separated from the actual sterilization process. The species active for sterilization are generated in the ionization phase and in the ensuing recombination and cooling phase and only the plasma processed gas formed therefrom is used, so that a direct plasma treatment does not take place. Thus, even thermally labile substances can be treated without problems.

US 2013/0142694 A1 describes a process of treating objects with a plasma processed gas and contacting water with such a gas yielding a sterilizing gas and a sterilizing solutions, wherein the sterilizing gas is sued to treat the object.

Irrespective of the method used for decontamination/disinfection/sterilization, a preceding cleaning step has a significant impact on the end result of the treatment. Usually, cleaning and decontamination/disinfection/sterilization are regarded as two separate steps, thus leading to scarce coordination between both processes. This often results in the use of excess amounts of sterilizing agents in order to achieve the desired grade of reduction of the microbial population.

One important problem underlying the present invention is to provide a rapid, inexpensive and safe method for obtaining clean and decontaminated/disinfected/sterile objects.

WO 2011/138463 A1 also describes a device that uses a plasma processed gas (PPG) for providing a sterilizing gas and/or a sterilizing liquid for the sterilization of objects. This device does not provide a mechanism for cleaning of the objects to be treated prior to the sterilization step.

Previously, plasma-generated sterilizing liquids have been produced in the range of several milliliters. However, for applications in the food industry, several thousand liters are necessary.

These problems are solved by the subject-matter of the independent claims.

### Summary of the invention

According to an aspect of the invention, a process of treating objects, particularly of cleaning and decontaminating and/or disinfecting and/or sterilizing objects, comprising the steps of
a. providing a plasma processed gas (PPG); and
b. contacting a liquid with the plasma processed gas (PPG), thus yielding a sterilizing gas and/or a sterilizing liquid; is provided, characterized in that
c. the object to be treated is contacted with the sterilizing liquid in a first treatment step; and
d. subsequently, the object to be treated is contacted with a treatment gas in a second treatment step, wherein the treatment gas comprises the sterilizing gas and/or the plasma processed gas (PPG).

### Terms and definitions

In the context of the present specification, the terms cleaning, decontamination, disinfection and disinfection signify a reduction of the microbial population at the specimen.

In the context of the present specification, the term cleaning signifies the process of removing residue such as dirt and part of the microbial population from an object by treatment with a liquid.

In the context of the present specification, the term decontamination signifies a reduction of the microbial population.

In the context of the present specification, the term disinfection signifies an inactivation of the microbial population by factor ≥ 10⁵.

In the context of the present specification, the term sterilization signifies an inactivation of the microbial population by factor ≥ 10⁶.

In the context of the present specification, the nature of the objects to be treated defines the desired and/or feasible degree of reduction of the microbial population.

The characteristics of the sterilizing agents and the treatment times are adjusted to the nature of the objects to be treated and the desired/feasible degree of reduction of the microbial population.

### Detailed description of the invention

According to an aspect of the invention, a process of treating objects, particularly of cleaning and decontaminating and/or disinfecting and/or sterilizing objects, comprising the steps of
a. providing a plasma processed gas (PPG); and
b. contacting a liquid with the plasma processed gas (PPG), thus yielding a sterilizing gas and/or a sterilizing liquid; is provided, characterized in that
c. the object to be treated is contacted with the sterilizing liquid in a first treatment step; and
d. subsequently, the object to be treated is contacted with a treatment gas in a second treatment step, wherein the treatment gas comprises the sterilizing gas and/or the plasma processed gas (PPG).

By humidifying the plasma processed gas (PPG) with water in one or more of its states of aggregation, a partial reaction of water with components of the gas mixture is possible. The time of contact of the plasma processed gas (PPG) with water may vary from milliseconds to hours. This gas formed by reaction with water (sterilizing gas) and also the solution formed from the reaction of plasma processed gas (PPG) with water (sterilizing solution) may be used as sterilizing agents. The humidification of the plasma processed gas (PPG) with water may be carried out as far as saturation.

In certain embodiments, the liquid that is contacted with the plasma processed gas (PPG) is water.

In certain embodiments, said plasma processed gas (PPG) is provided by
a. generating a plasma gas from a process gas; and
b. generating NOx and/or free radicals.

In certain embodiments, said process gas is air.

In certain embodiments, said process gas is an industrial gas.

In certain embodiments, said process gas is a shielding gas, particularly helium, argon, carbon dioxide, oxygen, nitrogen or hydrogen.

In certain embodiments said process gas is a shielding gas used for foodstuff, particularly CO₂, N₂, N₂O or O₂ or a mixture thereof.

To provide the plasma processed gas (PPG), the process gas, preferably dry air, is passed through and partly ionized in a plasma source (for example of a microwave plasma) that preferably forms a volume plasma, wherein a hot plasma gas of the process gas, particularly of the dry air, with a gas temperature of at least 1200°C is formed, usually under atmospheric pressure. After the plasma gas emerges from the excitation zone of the plasma source, it is subjected to specific cooling to bring about the formation of a plasma processed gas (PPG) after a certain reaction time. Current practice is to specify, by numerical simulation, the cooling process as a function of the necessary starting species. Appropriate and known cooling mechanisms are applied to cool the plasma gas as required, but at least to below 400 °C. Generation of the plasma processed gas (PPG) is described in detail in WO 2011/138463 A1.

The process according to any one of the above claims, characterized in that said plasma processed gas (PPG) comprises as a reactive species NOx and/or free radicals.

In certain embodiments, said plasma gas contains NO, which is oxidized to NO₂.

In certain embodiments, said plasma gas contains NO, which is oxidized to NO₂ at temperatures below 400 °C.

In certain embodiments, said plasma processed gas (PPG) has an NO₂ content of at least 0.5‰.

In certain embodiments, said plasma processed gas (PPG) has an NO₂ content of 0.5‰ to 5%.

In certain embodiments, said plasma processed gas (PPG) has an NO₂ content of 0.5‰ to 0.29%.

In certain embodiments, the treatment gas is the plasma processed gas (PPG).

Benefits of the plasma processed gas (PPG) are its antimicrobial effect and its drying effect with respect to the previously used sterilizing liquid. In addition, the plasma processed gas (PPG) reacts with the thin film of sterilizing liquid remaining after the cleaning step, thus generating additional antimicrobial agents directly at the object to be treated.

In certain embodiments, the treatment gas is the sterilizing gas.

Benefits of the plasma processed gas (PPG) are its very strong antimicrobial effect and its drying effect, which can be adjusted with the degree of humidification.

In certain embodiments, the sterilizing liquid exhibits a temperature of 0.1 °C to 100 °C, particularly 4° C to 60 °C, more particularly 10 °C to 40 °C, even more particularly approx. 25 °C.

In certain embodiments, the treatment gas exhibits a temperature of -80 °C to 400 °C, particularly -30 °C to 200 °C, more particularly 0 °C to 100 °C, even more particularly 8 °C to 40 °C.

In certain embodiments, the treatment gas exhibits a temperature of -100 °C to 5 °C, particularly -80 °C to 0 °C, more particularly -65 °C to -10 °C, even more particularly -40 °C to -20 °C.

The water contained in foodstuffs comprises mineral salts, carbohydrates, organic acids and other solvents. Thus, the freezing point is lowered to -0.5 °C to -3 °C. Crystallization of the water leads to an enrichment of these agents in the remaining water, further lowering the freezing point. The water freezes gradually over a wide temperature range until the eutectic point at -62 °C. At higher temperatures, the remaining liquid water enables enzyme activity, leading to a limited storage life even of deep-frozen foodstuffs. To guarantee long storage life temperatures significantly below the eutectic point are needed.

In certain embodiments, the treatment gas exhibits a temperature of 20 °C to 400 °C, particularly 30 °C to 300 °C, more particularly 60° C to 250 °C.

In general, higher temperatures lead to faster and more efficient decontamination/disinfection/sterilization. Some thermo-labile goods however require the use of lower temperature ranges.

In certain embodiments, the object to be treated is contacted with the sterilizing liquid for 2 seconds to 60 minutes, particularly for 10 seconds to 10 minutes; and subsequently contacted with the process gas for 2 seconds to 60 minutes, particularly for 10 seconds to 10 minutes.

In certain embodiments, a non-frozen object is contacted with the sterilizing liquid for 2 seconds to 60 minutes and subsequently contacted with the treatment gas for 2 seconds to 60 minutes, wherein the treatment gas exhibits a temperature of -100 °C to 0 °C, yielding a deep-frozen object after the treatment.

In certain embodiments, a deep-frozen object is contacted with the sterilizing liquid for 2 seconds to 60 minutes and subsequently contacted with the treatment gas for 2 seconds to 60 minutes, wherein the treatment gas exhibits a temperature of -100 °C to 0 °C, keeping the object deep-frozen during the treatment.

In certain embodiments, a deep-frozen object is contacted only with the treatment gas for 2 seconds to 60 minutes, wherein the treatment gas exhibits a temperature of -100 °C to 0 °C, keeping the object deep-frozen during the treatment.

The objects to be treated are exposed to the action of the sterilizing solution for a specified period. For this purpose, either the sterilizing solution is passed over the objects to be treated or the objects are contained in a process chamber, into which the sterilizing solution is admitted.

The effect of the sterilizing solution is both a mechanical/hydrodynamic cleaning effect and an antimicrobial effect due to its sterilizing properties.

Following removal of the sterilizing liquid, a thin film of the sterilizing liquid will remain on the objects to be treated.

The objects to be treated are now subsequently exposed to the action of the treatment gas for a specified period. For this purpose, either the treatment gas is passed over the objects to be treated or the objects are contained in a process chamber, into which the treatment gas is admitted.

When the treatment gas is admitted to the objects, the sterilizing properties of the remaining liquid are potentiated, due to the contact between treatment gas and remaining film of sterilizing liquid.

The humidification of the treatment gas may be adjusted to a desired level.

In addition to the decontaminating/disinfecting/sterilizing effect of the treatment gas, a drying effect is also achieved, leading to removal of all residual liquid derived from the sterilizing liquid (sterilizing liquid residue) of the previous cleaning step. This is of great importance for the end result of the cleaning and decontamination/disinfection/sterilization processes, since residual moisture from the cleaning step can result in new contamination.

After the specified time of action, the process chamber is purged with sterile air or in case of sterile packaged products with ambient air until the air quality conforms to the permissible workplace concentrations. The treatment gas may be disposed of by known appropriate measures, such as are achieved, for example, by using a gas scrubber or absorber material.

To improve the effect in narrow lumens and gaps, the process chamber may be evacuated before admission of the treatment gas. By admitting the treatment gas until atmospheric pressure and above is established, even narrow lumens and gaps can be reached. By applying a pressure change multiple times during the process of action of the treatment gas (pressure-alternation technology), the decontamination/disinfection/sterilization process may be accelerated and the functional reliability improved, especially in cavities. By a larger number of pressure cycles, the pressure difference of the cycles may be reduced. In this way, pressure sensitive items may be treated.

For treatment of objects that consist entirely or partly of cavities, the sterilizing liquid and the treatment gas may be admitted directly into or through the cavities.

The treatment gas may also be generated by passing air through the sterilizing solution, which has been prepared beforehand, resulting from the reaction of the plasma processed gas (PPG) with water, whereupon the humidified gas formed in this way is used for sterilization.

It is also possible to replace the plasma processed gas (PPG) mixture by a corresponding synthetic gas mixture.

The advantages of the inventive method compared with the prior art are based in particular on the fact that cleaning process and the decontamination/disinfection/sterilization process are coordinated and exercised in quick succession, yielding several synergistic effects.
1) Cleaning of the objects prior to sterilization highly increases the grade of sterility achieved by the sterilization process.
2) The contact between the freshly admitted treatment gas and the remaining film of sterilizing liquid potentiates the effect of antimicrobial agents in the liquid.
3) Drying of the objects after cleaning and decontamination/disinfection/sterilization prevents new contamination due to residual moisture.

In this way, extremely short and highly effective treatment times are achieved for the objects to be treated. Advantages are a reduction in the amount of sterilizing agents needed, reduced treatment times, reduced material damage, reduced costs, easier and faster handling, decreased risk of contamination.

Efficient and gentle treatment of objects is possible, even if they exhibit narrow gaps or capillaries. This method is suitable for thermo-labile goods like food and pharmaceutical products and their processing machines, as well as for medical products and packaging materials.

The sterilizing agents can be produced inexpensively in the exact amounts needed from safe and accessible materials (water, air and electrical energy). Thus, no storage, handling and/or transport of hazardous substances is required. The sterilizing agents can be produced at any time, thus enabling the immediate decontamination/disinfection/sterilization of objects when necessary, without the need for intensive preparation and planning-ahead.

The method can be performed by a device for the treatment of objects, particularly for the cleaning and decontamination and/or disinfection and/or sterilization of objects, comprising
a. an equipment for providing a plasma processed gas (PPG) (1); and
b. an equipment for supplying a liquid (3);
characterized in that said device comprises an equipment (2) able to contact the liquid with the plasma processed gas (PPG), yielding said sterilizing liquid and said sterilizing gas [humidification step].

The humidification of the treatment gas may be adjusted to a desired level. If a water bath is used for contacting the plasma processed gas (PPG) with the liquid, the degree of humidification can be adjusted by controlling the contact time of the plasma processed gas (PPG) with the liquid or by controlling the temperature of the plasma processed gas (PPG) or liquid. In a water bath, contact of the plasma processed gas (PPG) with the liquid can be accomplished by bubbling the treatment gas into the liquid via a frit or by contact of the plasma processed gas (PPG) with liquid at the liquid surface. If an injection device, a vaporizer or an ultrasonic nebulizer is used for contacting the plasma processed gas (PPG) with the liquid, the degree of humidification can be adjusted by controlling the flow of liquid.

The equipment for providing a plasma processed gas (PPG) comprises a supply unit for the process gas, particularly air, a plasma source for generating a plasma gas, a power supply for the plasma source, a compressor and a device in which the plasma gas is cooled and oxidized.

In certain embodiments, the equipment for generation of the plasma gas is operated at atmospheric pressure.

In certain embodiments, the equipment for contacting the plasma processed gas (PPG) with water is a pump.

In certain embodiments, the equipment for contacting the plasma processed gas (PPG) with water is a water jet pump.

In certain embodiments, the equipment for contacting the plasma processed gas (PPG) with water comprises a water jet pump and a sealed-off reaction line 202, wherein the sealed-off reaction line exhibits a length of 2 m to 100 m and/or a diameter of 12 mm to 51 mm.

In certain embodiments, the equipment for contacting the plasma processed gas (PPG) with water comprises a water jet pump and a sealed-off reaction line 202, wherein the sealed-off reaction line exhibits a length of 5 m to 15 m and/or a diameter of 25 mm to 39 mm.

In certain embodiments, the equipment for contacting the plasma processed gas (PPG) with water comprises a water jet pump and a sealed-off reaction line 202, wherein the sealed-off reaction line exhibits a length of around 12 m and a diameter of 32 mm.

Fast introduction of large volumes of plasma processed gas (approx. 100 l/min) generates a high back pressure in the equipment for contacting the plasma processed gas (PPG) with the liquid. This would require a high pressure in the equipment for providing the plasma processed gas (PPG). Using a water jet pump for contacting the plasma processed gas (PPG) with the liquid establishes a way to overcome the high back pressure. Thus, no high pressure in the equipment for providing the plasma processed gas (PPG) is required and plasma gas and plasma processed gas (PPG) can be generated at atmospheric pressure.

In certain embodiments, the device comprises a second pump 4, particularly a circulation pump, to return the sterilizing liquid to the equipment 2, for an additional round of contact with the plasma processed gas (PPG).

This circulation process allows increasing the concentration of the antimicrobial components of the sterilizing liquid continuously to the necessary concentration.

In certain embodiments, the device comprises a mechanism to subsequently contact the objects to be treated with said sterilizing liquid and said treatment gas in quick succession and at the same location.

Thus, the need for transport of objects between different devices for the cleaning and decontamination/disinfection/sterilization processes is eliminated.

In certain embodiments, said mechanism comprises appropriate lines and valves.

In certain embodiments, the device provides a mechanism for contacting the objects to be treated with the plasma processed gas (PPG), comprising a line 1 and a valve 502.

In certain embodiments, the device provides a mechanism for contacting the objects to be treated with the sterilizing liquid, comprising a line 305 and a valve 501.

In certain embodiments, the device provides a mechanism for contacting the objects to be treated with the sterilizing gas, comprising a line 301 and a valve 503.

In certain embodiments, the device provides a mechanism for subsequently contacting the objects to be treated with the sterilizing liquid and the plasma processed gas (PPG), comprising respective lines 305 and 1 and valves 501 and 502.

In certain embodiments, the device provides a mechanism for subsequently contacting the objects to be treated with the sterilizing liquid and the sterilizing gas, comprising respective lines 305 and 301 and valves 501 and 503.

In certain embodiments, the device provides a mechanism for subsequently contacting the objects to be treated with the sterilizing liquid, the sterilizing gas and the plasma processed gas (PPG) in any order, comprising respective lines 305, 301 and 1 and valves 501, 503 and 502.

After contact with the objects to be treated, the treatment gas may be reused and returned to the device for providing the plasma processed gas 1.

After contact with the objects to be treated, the sterilizing liquid may be reused and returned to the device for providing the liquid 2.

The device may be expediently supplemented by additionally disposing a vacuum pump or an air-circulation unit.

The advantages of the device compared with the prior art are based in particular on the fact that a single device enables the provision of both a sterilizing liquid that can be used for cleaning and a treatment gas that can be used for decontamination/disinfection/sterilization and drying of objects. The sterilizing agents are provided in sufficient amounts for use of the device in the medical business for sterilization of medical equipment and by the food industry.

Thus, an inexpensive method for cleaning and decontamination/disinfection/sterilization of objects is provided, wherein the species having the cleaning and decontaminating/disinfecting/sterilizing effect are generated preferably using air, water and electrical energy.

The invention will be explained hereafter on the basis of exemplary embodiments.

### Description of the figures

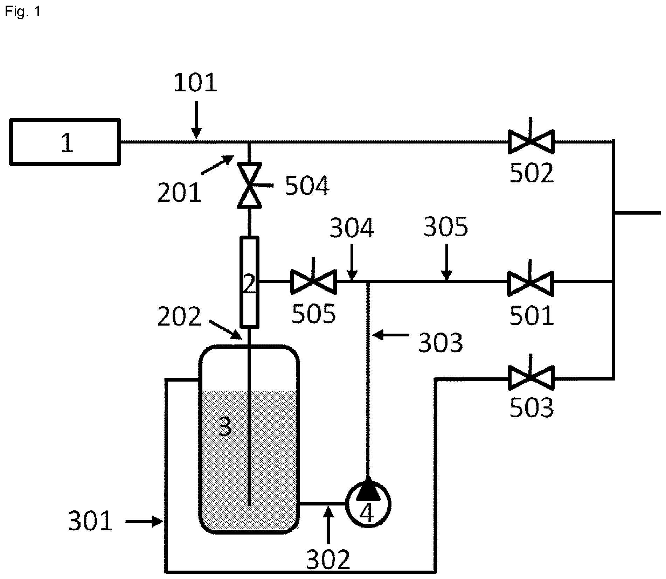

Figure 1 shows a diagram of the device.
   A plasma processed gas (PPG) is generated by device 1 comprising a microwave generator, plasma source, compressor and cooling/oxidation device. The plasma processed gas (PPG) can be directly delivered to the objects to be treated via line 101 and valve 502. Alternatively, the plasma processed gas (PPG) can be delivered to pump 2 via line 201. Admission to the pump 2 is controlled via valve 504, which is operated together with valve 502. A liquid is provided in a container 3 and delivered to the pump 2 via line 302, a recirculation pump 4, and lines 303 and 304. Admission to the water jet pump is controlled via valve 505. In pump 2 and pump line 202, which provides a sealed-off reaction line, the plasma processed gas (PPG) is contacted with water. A sterilizing liquid and a sterilizing gas are generated and delivered to the container 3. The sterilizing gas is delivered from the container 3 to the objects to be treated via pressure relief line 301 and valve 503. The sterilizing liquid can be delivered from the container 3 back to the pump 2 to increase the content of antimicrobial agents. Alternatively, the sterilizing water can be delivered to the objects to be treated via line 302, the recirculation pump 4, lines 303 and 305 and valve 501, which is operated together with valve 505.

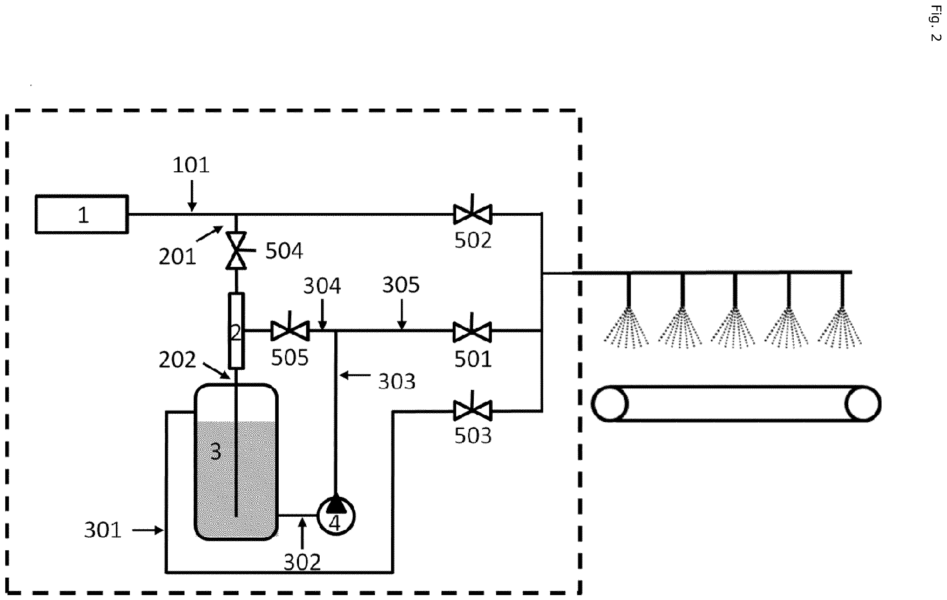

Figure 2 depicts an application of the device according to figure 1 for sterilization of goods, e.g. salad.

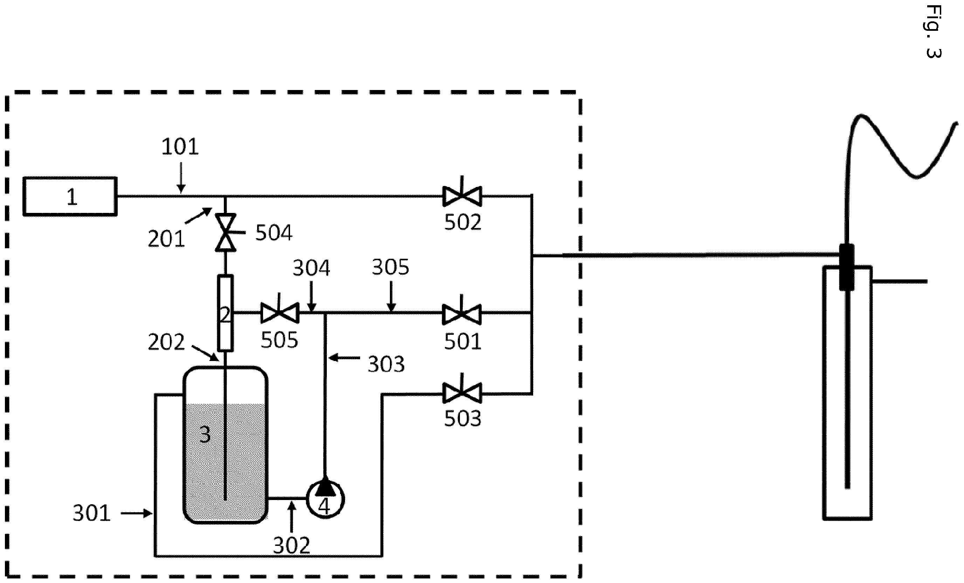

Figure 3 depicts an application of the device according to figure 1 for sterilization of medical equipment, e.g. endoscopes.

**Reference numerals**

| # | description |
|---|---|
| 1 | device for providing plasma-processed gas (PPG) |
| 101 | line for plasma processed gas (PPG) |
| 2 | pump for contacting PPG and liquid |
| 201 | line for delivering plasma processed gas (PPG) to pump (2) |
| 202 | pump line and reaction device for gas-liquid-interaction |
| 203 | reflux line for sterilizing liquid, input for pump (2) |
| 3 | container for liquid and gas |
| 301 | line for sterilizing gas / pressure relief line |
| 302 | line for delivering liquid to pump (2) (circulation) |
| 303 | line for delivering liquid to pump (2) (circulation) |
| 304 | line for delivering liquid to pump (2) (circulation) |
| 305 | line for delivering sterilizing liquid to objects |
| 4 | recirculation pump |
| 501 | valve for providing sterilizing liquid |
| 502 | valve for providing plasma processed gas (PPG) |
| 503 | valve for providing sterilizing gas / pressure relief valve |
| 504 | valve for controlling gas flow to pump (2) |
| 505 | valve for controlling liquid flow to pump (2) |

## Claims

1. A process of treating objects, particularly of cleaning and decontaminating and/or disinfecting and/or sterilizing objects, comprising the steps of
a. providing a plasma processed gas (PPG); and
b. contacting a liquid with said plasma processed gas (PPG), wherein in particular said liquid is water, thus yielding a sterilizing gas and a sterilizing liquid;
**characterized in that**
c. the object to be treated is contacted with said sterilizing liquid in a first treatment step; and
d. subsequently, the object to be treated is contacted with a treatment gas in a second treatment step, wherein said treatment gas comprises said plasma processed gas (PPG) and/or said sterilizing gas.

2. The process according to claim 1, **characterized in that** said plasma processed gas (PPG) is provided by
a. generating a plasma gas from a process gas, particularly air; and
b. generating NOx and/or free radicals.

3. The process according to any one of the above claims, **characterized in that** said treatment gas exhibits a temperature of -80 °C to 400 °C.

4. The process according to any one of the above claims, **characterized in that** the object to be treated is contacted with said sterilizing liquid for 2 seconds to 60 minutes, particularly for 10 seconds to 10 minutes and subsequently contacted with said process gas for 2 seconds to 60 minutes, particularly for 10 seconds to 10 minutes.

5. The process according to any one of the above claims, **characterized in that** said treatment gas is said sterilizing gas.

## Patentansprüche

1. Ein Verfahren zum Behandeln von Gegenständen, insbesondere zum Reinigen und Dekontaminieren und/oder Desinfizieren und/oder Sterilisieren von Gegenständen, umfassend der Schritte
a. Bereitstellen eines plasmavearbeiteten Gases (PPG); und
b. Kontaktieren einer Flüssigkeit mit besagtem plasmavearbeiteten Gas (PPG), wobei insbesondere besagte
Flüssigkeit Wasser ist, somit ergebend ein sterilisierendes Gas und eine sterilisierende Flüssigkeit;
**dadurch gekennzeichnet, dass**
c. der zu behandelnde Gegenstand in einem ersten Behandlungsschritt mit besagter sterilisierender Flüssigkeit kontaktiert wird; und
d. anschließend der zu behandelnde Gegenstand mit einem Behandlungsgas in einem zweiten Behandlungsschritt kontaktiert wird, wobei besagtes Behandlungsgas besagtes plasmavearbeitete Gas (PPG) und/oder besagtes sterilisierende Gas umfasst.

2. Das Verfahren gemäß Anspruch 1, **dadurch gekennzeichnet, dass** besagtes plasmaverarbeitete Gas (PPG) bereitgestellt ist durch
a. erzeugen eines Plasmagases aus einem Verfahrensgas, insbesondere Luft; und
b. erzeugen von NOx und/oder freien Radikalen.

3. Das Verfahren gemäß einem der oben genannten Ansprüche, **dadurch gekennzeichnet, dass** besagtes Behandlungsgas eine Temperatur von -80 °C bis 400 °C aufweist.

4. Das Verfahren gemäß einem der oben genannten Ansprüche, **dadurch gekennzeichnet, dass** der zu behandelnde Gegenstand mit besagter sterilisierender Flüssigkeit für 2 Sekunden bis 60 Minuten kontaktiert wird, insbesondere für 10 Sekunden bis 10 Minuten und anschließend mit dem Verfahrensgas für 2 Sekunden bis 60 Minuten, insbesondere für 105 Sekunden bis 10 Minuten kontaktiert wird.

5. Das Verfahren gemäß einem der oben genannten Ansprüche, **dadurch gekennzeichnet, dass** besagtes Behandlungsgas besagtes sterilisierende Gas ist.

## Revendications

1. Procédé de traitement d'objets, en particulier de nettoyage et de décontamination et/ou de désinfection et/ou de stérilisation d'objets, comprenant les étapes :
a. la fourniture d'un gaz traité par plasma (plasma processed gas - PPG) ; et
b. la mise en contact d'un liquide avec ledit gaz traité par plasma (PPG), ledit liquide étant en particulier de l'eau, ce qui permet d'obtenir un gaz stérilisant et un liquide stérilisant ;
**caractérisé en ce que**
c. l'objet à traiter est mis en contact avec ledit liquide stérilisant dans une première étape de traitement ; et
d. ensuite, l'objet à traiter est mis en contact avec un gaz de traitement dans une deuxième étape de traitement, ledit gaz de traitement comprenant ledit gaz traité par plasma (PPG) et/ou ledit gaz stérilisant.

2. Procédé selon la revendication 1, **caractérisé en ce que** ledit gaz traité par plasma (PPG) est fourni par :
a. génération d'un gaz plasma à partir d'un gaz de procédé, en particulier d'air ; et
b. génération de NOx et/ou de radicaux libres.

3. Procédé selon l'une quelconque des revendications précédentes, **caractérisé en ce que** ledit gaz de traitement présente une température de -80 °C à 400 °C.

4. Procédé selon l'une quelconque des revendications précédentes, **caractérisé en ce que** l'objet à traiter est mis en contact avec ledit liquide stérilisant pendant 2 secondes à 60 minutes, en particulier pendant 10 secondes à 10 minutes, et ensuite mis en contact avec ledit gaz de procédé pendant 2 secondes à 60 minutes, en particulier pendant 10 secondes à 10 minutes.

5. Procédé selon l'une quelconque des revendications précédentes, **caractérisé en ce que** ledit gaz de traitement est ledit gaz stérilisant.
